# EUROPEAN PATENT SPECIFICATION

(11) **EP 3 787 632 B1**
(45) Date of publication and mention of the grant of the patent: **25.05.2022**
(21) Application number: 19720629.5
(22) Date of filing: 01.05.2019
(51) Int. Cl.: A61K 31/565, A61K 45/06, A61P 35/00

(54) **TREATMENT REGIMEN FOR THE TREATMENT OF ADVANCED OESTROGEN RECEPTOR POSITIVE BREAST CANCER**
DOSIERUNGSSCHEMA ZUR BEHANDLUNG VON FORTGESCHRITTENEM ÖSTROGENREZEPTOR-POSITIVEM BRUSTKREBS
REGIME POSOLOGIQUE POUR SON UTILISATION DANS LE TRAITEMENT DU CANCER DU SEIN POSITIF AUX RÉCEPTEURS DES OESTROGÈNES AVANCÉ

(30) Priority: 02.05.2018 EP 18170382
(43) Date of publication of application: 10.03.2021
(73) Proprietor: Estetra SPRL, 4000 Liège (BE)
(72) Inventor: COELINGH BENNINK, Herman Jan Tijmen, 3707 BK Zeist (NL); VERHOEVEN, Carole, 3707 BK Zeist (NL)
(74) Representative: Nederlandsch Octrooibureau
(86) International application number: PCT/EP2019/061142
(87) International publication number: WO 2019/211322

(56) References cited:
- WO-A1-2004/103377
- WO-A2-2009/138780
- US-A1- 2015 157 606
- COELINGH BENNINK HERJAN J T ET AL: "Clinical effects of the fetal estrogen estetrol in a multiple-rising-dose study in postmenopausal women", MATURITAS, ELSEVIER, AMSTERDAM, NL, vol. 91, 23 June 2016 (2016-06-23), pages 93-100, XP029649879, ISSN: 0378-5122, DOI: 10.1016/J.MATURITAS.2016.06.017 cited in the application
- C. F. SINGER ET AL: "Antiestrogenic effects of the fetal estrogen estetrol in women with estrogen-receptor positive early breast cancer", CARCINOGENESIS., vol. 35, no. 11, 5 July 2014 (2014-07-05), pages 2447-2451, XP055511590, GB ISSN: 0143-3334, DOI: 10.1093/carcin/bgu144 cited in the application
- H J T COELINGH BENNINK: "ESTETROL FOR HRT WITH SPECIAL EMPHASIS ON BREAST CANCER", MATURITAS, vol. 71, no. 1, 14 September 2012 (2012-09-14), page S17, XP055599604, DOI: https://doi.org/10.1016/S0378-5122(12)7007 3-7
- WEI YUE ET AL: "Pro-Apoptotic Effects of Estetrol on Long-Term Estrogen-Deprived Breast Cancer Cells and at Low Doses on Hormone-Sensitive Cells", BREAST CANCER : BASIC AND CLINICAL RESEARCH, vol. 13, 15 January 2019 (2019-01-15), XP055599598, New Zealand ISSN: 1178-2234, DOI: 10.1177/1178223419844198
- HERJAN J T COELINGH BENNINK ET AL: "A Dose-Escalating Study With the Fetal Estrogen Estetrol in Healthy Men", JOURNAL OF CLINICAL ENDOCRINOLOGY AND METABOLISM, vol. 103, no. 9, 1 September 2018 (2018-09-01), pages 3239-3249, XP055511617, US ISSN: 0021-972X, DOI: 10.1210/jc.2018-00147

## Description

The present invention relates to the field of breast cancer treatment. More particularly, the invention relates to products for use in the treatment of advanced estrogen receptor positive breast cancer in a subject who has been treated with an estrogen activity suppressor selected from a selective estrogen receptor modulator (SERM), an aromatase inhibitor and an anti-estrogen, said treatment comprising administration of an estetrol component selected from estetrol, prodrugs of estetrol and combinations thereof, within 12 weeks after the treatment with an estrogen activity suppressor has been discontinued.

### Background art

Breast cancer is one of the leading causes of cancer mortality among Western women, and is predicted to become a leading cause of cancer death in Oriental women in countries such as Japan in the near future. The American Cancer Society estimates that 1 in 9 women face a lifetime risk of this disease, which will prove fatal for about one-quarter of those afflicted with the disease. Breast tumours are known to be estrogen-sensitive, meaning that the formation and growth of such tumours is stimulated by estrogens such as 17beta-estradiol. 17beta-estradiol is an estrogen that is endogenous to the human body and that is found in both females and males. Estrogens are known to increase the risk of breast tumours by inducing an estrogen receptor (ER) mediated increase in the frequency of breast cell division (proliferation). Cell division is essential in the complex process of genesis of human cancer since it per se increases the risk of genetic error, particularly genetic errors such as inactivation of tumour suppressor genes.

An important element of the treatment of estrogen-sensitive tumours is the suppression of undesirable estrogen-induced effects. Estrogen induced effects can be suppressed or even eliminated by administering an estrogen activity suppressor such as a selective estrogen receptor modulator (SERM), an aromatase inhibitor or an anti-estrogen.

A commonly used therapy to block estrogen receptor sites involves the administration of anti-estrogens. Anti-estrogens are a class of chemicals which prevent estrogens from eliciting their full response in target tissues. An example of such a compound is fulvestrant, which is a pure anti-estrogen since it degrades the estrogen receptor.

Selective estrogen receptor modulators (SERMs) are another class of estrogen activity suppressors that are commonly used in the treatment of estrogen-sensitive cancers. Tamoxifen (TAM) is an example of a SERM. Unlike anti-estrogens, SERMs exhibit both estrogen antagonist and agonist properties.

Aromatase Inhibitors (Als) work by blocking the production of estrogens. There are two types of aromatase inhibitors approved to treat breast cancer: irreversible steroidal inhibitors, such as exemestane, which forms a permanent and deactivating bond with the aromatase enzyme (the enzyme responsible for the synthesis of estrogens), and non-steroidal inhibitors, such as anastrozole and letrozole which inhibit the synthesis of estrogen via reversible competition for the aromatase enzyme.

Treatment of breast cancer by administering an estrogen activity suppressor (endocrine therapy) is often highly effective, but its usefulness is limited by common intrinsic and acquired resistance. Multiple mechanisms responsible for endocrine resistance have been proposed and include deregulation of various components of the ER pathway itself, alterations in cell cycle and cell survival signaling molecules, and the activation of escape pathways that can provide tumours with alternative proliferative and survival stimuli.

Thus, despite the benefits of using estrogen activity suppressor in the treatment of ER-positive breast cancer, resistance to treatment eventually occurs in a large number of patients (A. A. Larionov and W. R. Miller, Future Oncology, vol. 5, no. 9, pp. 1415-1428, 2009). Clinically, resistance can manifest itself as relapse or cancer recurrence during or after completion of drug therapy, following surgery or in rare cases after complete pathological response (elimination of all cancer tissue). Alternatively, in the neoadjuvant setting, resistance can be observed as clinical progression of primary disease, usually constituting an increase in primary tumour size or disease spread to regional nodes or beyond to more distant metastatic sites. Pathological changes such as increased tumour grade or increased proliferation are indicators of potential resistance to therapy. In the neoadjuvant setting, resistance occurs as either a primary lack of response early in treatment, implying innate resistance, or later following a period of response, suggesting acquired resistance. It has been suggested that as many as 40-50% of all ER-positive patients treated with estrogen activity suppressor will eventually relapse (C. X. Ma, C. G. Sanchez, and M. J. Ellis, Oncology, vol. 23, no. 2, pp. 133-142, 2009).

The current clinical practice, when confronted with a breast tumour having developed resistance to estrogen activity suppressor, is to initiate treatment with chemotherapy drugs. These powerful drugs, however, have very detrimental side effects. Therefore, there is a great need for alternative treatments for advanced estrogen receptor positive breast cancers that have less damaging side effects.

Singer et al. (Antiestrogenic effects of the fetal estrogen estetrol in women with estrogen-receptor positive early breast cancer, Carcinogenesis (2014), 35, 2447-2451) describe a study in which the effect of 14 days preoperative treatment with 20 mg E4 per day on tumor proliferation markers, sex steroid receptor expression and endocrine parameters was investigated in a prospective, randomized, placebo-controlled, preoperative window trial in 30 pre- and postmenopausal women with estrogen-receptor positive early breast cancer

WO 2004/103377 describes the use of estetrol in the treatment or prevention of musculoskeletal pain in a mammal receiving administration of an estrogen suppressant selected from the group consisting of aromatase inhibitors, GnRH analogues, cyclo-oxygenase 2 (COX-2) inhibitors, 17ss-hydroxysteroid dehydrogenase type 1 inhibitors, progestogens, anti-estrogens and combinations thereof, said treatment comprising administering estetrol in an effective amount to prevent said musculoskeletal pain,

Coelingh Bennink et al. (Clinical effects of the fetal estrogen estetrol in a multiple-rising-dose study in postmenopausal women, Maturitas (2016), 91, 93-100) describes the effects of estriol on growth, gene expression and estrogen response element activation in human breast cancer cell lines. It was found that estriol acted as a potent estrogen and exerted a mitogenic effect on T-47D and MCF-7 cells at concentrations of 10-9 M (288 pg/ml) and higher. The authors conclude: "Like E2 (estradiol), low levels of E3 were able to trigger a robust estrogenic response in breast cancer cells. Thus, our data suggest caution regarding use of E3 by breast cancer survivors".

### Summary of the invention

The invention is defined by the claims. Any subject-matter falling outside the scope of the claims is provided for information purposes only.

Any references in the description to methods of treatment, diagnosis or surgery refer to the compounds, pharmaceutical compositions and medicaments of the present invention for use in a method for treatment, diagnosis or surgery of the human or animal body by therapy.

The present invention provides a useful alternative therapy for treating advanced estrogen receptor positive breast cancers that have become resistant to an estrogen activity suppressor or for treating advanced estrogen positive breast cancers in subjects who have rejected treatment with estrogen activity suppressor due to unacceptable side effects.

More particularly, the invention provides a treatment of advanced estrogen receptor positive breast cancer in a subject who has been treated with an estrogen activity suppressor selected from a selective estrogen receptor modulator (SERM), an aromatase inhibitor and an anti-estrogen, said treatment comprising administration of an estetrol component to said subject within 12 weeks after the treatment with an estrogen activity suppressor has been discontinued, said estetrol component being selected from estetrol, prodrugs of estetrol in the form of estetrol derivatives wherein the hydrogen atom of at least one of the hydroxyl groups has been substituted by an acyl radical of a hydrocarbon carboxylic, sulfuric acid, sulfonic acid or sulfamic acid of 1-25 carbon atoms, and combinations thereof.

The inventors have found that administration of an estetrol component to a subject suffering from breast cancer, and who has been treated with an estrogen activity suppressor, can have an unexpected favourable impact on tumour progression, and in some cases may even induce tumour regression. In addition, due to its estrogenicity, administration of the estetrol components to these subjects has an advantageous effect on Quality Of Life (QOL). Thus, treatment with the estetrol component can suitably be used to delay treatment with chemotherapy drugs and to eliminate hypoestrogenicity that was caused by the preceding treatment with estrogen activity suppressor.

This surprising finding stems from the observation by the inventors that an estetrol component can be administered at high doses to breast cancer patients without generating the unacceptable side effects usually observed for high doses of other estrogens. Furthermore, the treatment of the invention has a positive impact on the hypo-estrogenic side effects induced by previous treatment with estrogen activity suppressors. Examples of undesirable side-effect that can be remedied by the present treatment include mood disturbances (depression/irritability), hot flushes, arthralgia, vulvovaginal complaints, sleep disturbances, cognition problems, memory loss and bone loss.

The present treatment may employ oral, mucosal (such as sublingual, sublabial, buccal, intranasal), transdermal, parenteral (such as i.v.) or subcutaneous administration of the estetrol component.

### Detailed description of the invention

The invention is defined by the claims. Any subject-matter falling outside the scope of the claims is provided for information purposes only.

Any references in the description to methods of treatment, diagnosis or surgery refer to the compounds, pharmaceutical compositions and medicaments of the present invention for use in a method for treatment, diagnosis or surgery of the human or animal body by therapy.

### Definitions

The term "estetrol component", as used herein, encompasses estetrol as well as prodrugs of estetrol in the form of estetrol esters that are estetrol derivatives wherein the hydrogen atom of at least one of the hydroxyl groups has been substituted by an acyl radical of a hydrocarbon carboxylic, sulfuric acid, sulfonic acid or sulfamic acid of 1-25 carbon atoms.

The term "estetrol" refers to estra-1,3,5(10)-triene-3,15α,16α,17β-tetrol. The term estetrol also encompasses hydrates of this estrogen.

Whenever a "dose" or a "daily dose" is defined in terms of "estetrol equivalent", what is meant is that the dosage administered is equivalent to an orally administered estetrol monohydrate dose as specified. As used herein, the terms "advanced breast cancer" refers to locally advanced breast cancer (breast cancer that has progressed locally but there are no signs that the cancer has spread beyond the breast region) and/or metastatic breast cancer (breast cancer that has spread from its site of the origin to other parts of the body).

The qualification that a breast cancer has "acquired resistance to an estrogen activity suppressor" means that the treatment of the breast cancer with said estrogen activity suppressor is no longer effective as evidenced by a "progressive disease" categorization in accordance with the revised RECIST guideline (New response evaluation criteria in solid tumours: Revised RECIST guideline (version 1.1), Eur. J. Cancer (2009), 45, 228-247).

According to this guideline, the assessment is performed by comparing images of the tumour(s) at different stages during treatment. As further detailed in Section 4.3.1 of the revised RECIST guideline, the target lesions are classified into one of the following categories;
- complete response (CR);
- partial response (PR);
- progressive disease (PD);
- stable disease (SD).

In accordance with the present invention, administration of the estetrol component commences within 12 weeks after the treatment with an estrogen activity suppressor has been discontinued. The time period between discontinuation of the treatment with an estrogen activity suppressor and the beginning of the administration of the estetrol component equals the number of days that has lapsed between the first day on which the estrogen activity suppressor has not been administered according to the relevant protocol and the day on which the estetrol component is first administered.

### Methods of treatment

In accordance with a preferred embodiment, the present treatment comprises uninterrupted administration of doses of the estetrol component during a period of at least 2 weeks, preferably during a period or at least 4 weeks, in dosages equivalent to a daily oral dosage of 10 to 80 mg estetrol, more preferably equivalent to a daily oral dosage of 20 to 60 mg estetrol.

The amounts needed to be effective differ from individual to individual and are determined by factors such cancer type and stage, body weight, route of administration and the efficacy of the particular estrogenic substance used.

Administration of the estetrol component in accordance with the present invention preferably commences within 8 weeks, more preferably within 4 weeks after the treatment with an estrogen activity suppressor has been discontinued.

In one embodiment of the invention, the treatment comprises administration of the estetrol component to an oophorectomized or post-menopausal female subject who has decided to discontinue the treatment with an estrogen activity suppressor. Unacceptable symptoms of hypoestrogenicity are the main reason for subjects to discontinue treatment with estrogen activity suppressor. Administration of the estetrol component in accordance with the present invention quickly removes these unacceptable symptoms and generally has a favourable impact on the progression of the breast cancer.

In another, particularly preferred embodiment of the present invention, the treatment comprises administration of the estetrol component to a subject whose breast cancer has acquired resistance to said estrogen activity suppressor.

It is important to note that, prior to the present invention, the generally accepted practice for treating advanced estrogen receptor positive breast cancer that has become resistant to an estrogen activity suppressor was to avoid administering any kind of estrogen.

For example in a 2013 information booklet about menopause (accessible at: https://www.fda.gov:80/FDAgov/ForConsumers/ByAudience/ForWomen/ucm118627.htm), the FDA strongly warns against taking estrogen-containing medicines "*if you have or have had certain cancers such as breast cancer or uterine cancer*".

Similarly, the Patient Information Leaflet for the estrogen-only product Premarin states "*Do not take Premarin* [...] *if you have or have ever had breast cancer, or if you are suspected of having it*".

In a 2009 publication, Ellis et al. (Matthew J. Ellis et al ; 2009, JAMA, 302(7) : 774-780) report that estrogen treatment has an adverse effect on Quality of Life: as can be read in the Results section, under the paragraph "Quality of life analysis",
"*a significant increase in severity of side effects from baseline to follow-up was observed overall (0.47 to 0.80; P <0.001), but the change was not significantly different by treatment arm (0.47-0.70 in 6-mg arm vs. 0.46-0.92 in 30-mg arm; P =0.10)".*

Further, in the Discussion section of the publication, Ellis et al. state: "We also observed that intense estradiol side effects have an adverse effect on QOL which are mitigated by lowering the estradiol dose."

Thus it goes against this widely accepted doctrine to treat subjects with advanced estrogen receptor positive breast cancer with estrogenic estetrol component.

Without wishing to be bound by theory, the unexpected benefits (and absence of undesired estrogenic effects) are believed to be associated with the special properties of the estetrol component.

So far, estrogen administration in the context of breast cancer treatment has been constrained in terms of acceptable doses because of a number of side effects (such as, for example, nausea, or even thromboembolic and cardiovascular events in case of oral administration). The present applicant has demonstrated (among others, in Example 2), that an estetrol component can be administered in high doses without generating the side effects usually observed with the administration of high doses of other estrogens.

In addition, the applicant has demonstrated (among others, in Example 1), that administration of an estetrol component is surprisingly capable of delaying tumor growth.

Further, the present treatment is very effective in counteracting the hypo-estrogenism that is observed in breast cancer patients who have been treated with estrogen activity suppressors. The symptoms of hypo-estrogenism can be very severe and include mood disturbances (depression/irritability), hot flushes, arthralgia, vulvovaginal complaints, sleep disturbances, cognition problems, memory loss and bone loss.

A very important benefit of the second line treatment according to the present invention in comparison with ordinary second line treatment with chemotherapy drugs lies in the fact that contrary to the latter treatment, the present treatment does not have an adverse effect on Quality of Life (QOL) of the subject. As a matter of fact, as explained above, due to the fact that the present treatment counteracts hypo-estrogenism that is induced during the first line treatment, QOL of subjects is actually improved with the present treatment.

The quality of life of the subject is for example assessed with the Functional Assessment of Cancer Therapy -Breast (FACT-B) and Endocrine Subscale (FACT-ES) (version 4, see http://www.facit.org/FACITOrg/Questionnaires). More information on these assessments can be found in publications by Fallowfield et al. (British J. of Cancer, 2012, 106, p.1062-1067) and by Webster et al. (Health and Quality of Life Outcomes, 2003, 1, p.79).

Higher scores for the scales and subscales indicate better quality of life. A difference of 5 in the trial outcome index (TOI), a summation of the physical, functional and breast cancer concerns subscales, is considered to be the clinically relevant minimally important difference.

In a preferred embodiment, the quality of life of the subject treated by the therapy of the invention is accordingly improved during the course of the treatment by an increase of at least 5 in the TOI, preferably by an increase of at least 7 in the TOI, even more preferably an increase of at least 10 in the TOI.

In a particular embodiment of the invention, the estetrol component is administered during a treatment period of at least 8 weeks, preferably at least 24 weeks, more preferably at least 1 year.

In a further embodiment, the tumour burden is monitored during the treatment period at regular intervals. If tumour burden decreases (CR or PR under the RECIST criteria) or remains stable (SD under the RECIST criteria), the treatment with estetrol component is continued. If the tumour burden progresses (PD under the RECIST criteria) treatment with a chemotherapy drug is initiated.

According to a particularly preferred embodiment of the present treatment, administration of a chemotherapy agent is commenced when the monitoring shows that the tumour burden has increased. In an even more preferred embodiment, administration of the estetrol component is continued during administration of the chemotherapy agent, in particular if hypoestrogenic symptoms were improved during the earlier estetrol treatment.

In a particular embodiment the estetrol component is administered once daily in a convenient once-daily unit dose.

According to a particularly preferred embodiment, the present treatment comprises oral, sublingual, sublabial or buccal administration of the estetrol component. Most preferably, the treatment comprise oral administration of the estetrol component.

In the present treatment, the estetrol component is preferably administered in an amount effective to achieve an estetrol equivalent blood plasma trough concentration of at least 600 pg/mL, preferably of at least 1000 pg/mL, more preferably at least 1500 pg/mL, still more preferably at least 2000 pg/mL. As used herein, "trough levels" means the lowest concentration that a drug reaches before the next dose is administered.

Generally the resulting estetrol equivalent blood plasma trough levels do not exceed 20000 pg/mL, preferably it does not exceed 18000 pg/mL, more preferably it does not exceed 16000 pg/mL, still more preferably it does not exceed 14000 pg/mL.

As can be deducted from Example 2, a daily oral dosage of 10 mg estetrol results in a plasma trough concentration of estetrol above 1000 pg/mL.

The safety of the estetrol component is a key aspect of the present invention which makes it possible to administer this estrogenic component at much higher levels than other estrogens and thus renders the present treatment possible.

Yet another important aspect of the treatment of the invention is that, since estetrol itself does not bind to SHBG, changes in plasma levels of SHBG do not influence the availability of estetrol. This is by contrast to estradiol which binds to SHBG with high affinity of about 40 % (Hammond GL at al., Climacteric. 2008;11 Suppl 1:41-6). A direct consequence of this is that more of the administered estetrol is available by comparison with an estradiol-based treatment where a significant part of the administered drug is bound to SHBG.

For reasons of convenience and also to achieve high compliance rates, the present treatment preferably utilises administration intervals of 1 day, 1 week or 1 month. Regimens that employ once daily oral, sublingual, buccal or sublabial administration of the estetrol component are particularly preferred. Regimens that employ once daily oral administration of the estetrol component are most preferred.

### Patient population

The subject treated in accordance with the invention is preferably a human, especially a female. Said subject suffers from locally advanced and/or metastatic (herein, "advanced") breast cancer. Said breast cancer has an estrogen-receptor-positive status (ER+).

The present treatment is particularly effective if the subject is a post-menopausal female or a female who has undergone oophorectomy.

The subject undergoing treatment in accordance with the present invention preferably has previously been treated with tamoxifen and/or an aromatase inhibitor. In this embodiment, treatment with tamoxifen encompasses, for example, treatment with the drug Nolvadex^{™} and treatment with an aromatase inhibitor encompasses treatment with one or more of, for example, anastrosole (Arimidex^{™}), letrozole (Femara^{™}), exemestane (Aromasin^{™}) and/or aminoglutethimide (Orimeten^{™}).

In a particular embodiment, the subject has not received treatment with fulvestrant (7-alpha[9-(4,4,5,5,5-pentafluoropentylsulphinyl)nonyl]-estra-1,3,5(10)-triene-3, 17beta diol, marketed under the brand name Faslodex^{™}), within 6 months of start of the treatment according to the invention.

Another aspect of the invention relates to an oral dosage unit comprising an oral chemotherapy drug, together with 10-80 mg of the estetrol component, preferably 20-60 mg the estetrol component, wherein the oral chemotherapy drug is selected from capecitabine, cyclophosphamide, vinorelbine, methotrexate and combinations thereof.

Yet another aspect of the invention relates to a kit-of-parts comprising at least one dosage unit containing a chemotherapy drug and a plurality of oral dosage units containing 10-80 mg of the estetrol component, preferably 20-60 mg of the estetrol component, wherein the chemotherapy drug is selected from gemcitabine, docetaxel, paclitaxel, albumin-bound paclitaxel, cisplatin, carboplatin, doxorubicin, liposomal doxorubicin, epirubicin, eribulin, ixabepilone, cyclophosphamide, vinorelbine and combinations thereof. The chemotherapy drug is preferably provided in a dosage form that is suitable for intravenous administration.

It is particularly preferred that the kit-of-parts contains instructions to co-administer the at least one dosage unit containing the chemotherapy drug and the dosage units containing the estetrol component.

Examples of oral dosage units that may be employed in accordance with the present invention include tablets and capsules. Most preferably, the oral dosage unit is a tablet.

### Examples

### Example 1

In order to assess the potential of an estetrol component to inhibit the growth of estrogen deprived breast cancer cells, *in vitro* studies in long term estrogen deprived MCF7 breast cancer cells (LTED cells) were conducted. LTED cells are deprived of estrogen, making them a suitable *in vitro* model to study drug effects in women who have been using estrogen activity suppressors for a long time.

LTED cells were plated in 6-well plates at a density of 30,000 cells per well. The cells were maintained in phenol red free IMEM with 5% charcoal stripped FBS (DCC-FBS). On Day 3, the medium was replaced with fresh phenol red free IMEM with 5% DCC-FBS. The cells were then exposed to 6 different concentrations of estetrol ranging from 10⁻¹²M to 10⁻⁷ M or to ethanol as vehicle control. The final vehicle concentration was 0.1% ethanol for all experiments. Each treatment was made in duplicate.

On Day 5, the medium was changed and on Day 7 plates were subjected to a cell count analysis. The results are shown in Table 1.

**Table 1**

| | Cell number per well (×10⁵) |
|---|---|
| Control | 5.36 |
| 10⁻¹² M estetrol | 5.58 |
| 10⁻¹¹ M estetrol | 3.62 |
| 10⁻¹⁰ M estetrol | 0.63 |
| 10⁻⁹ M estetrol | 0.56 |
| 10⁻⁸ M estetrol | 0.59 |
| 10⁻⁷ M estetrol | 0.49 |

These results show that estetrol is able to inhibit the growth of LTED cells. Peak inhibition is observed at 10⁻¹⁰ M and no further inhibition is detected at higher estetrol concentrations.

### Example 2

For each group dosed at 2, 10, 20 and 40 mg estetrol daily (oral administration), 9 to 10 female patients were tested for their plasma content in estetrol at Day 7 and at Day 14. For each dose group, the average trough level of all patients across both days was calculated.

As illustrated in Table 2, the results show a nice dose linearity. In addition, this study has shown that estetrol at these high dose levels was well tolerated whereas comparable dosages of other estrogens cause significant side effects such as nausea, impacting negatively on the QOL.

**Table 2 - Trough and Cmax plasma levels of estetrol as a function of the administered oral dose**

| Daily estimated oral dose (mg) | Trough plasma level (pg/mL) | Cmax plasma level (pg/mL) |
|---|---|---|
| 2 | 240 | 1,300 |
| 10 | 1,500 | 9,500 |
| 20 | 1,950 | 23,500 |
| 40 | 5,070 | 82,700 |

### Example 3

A multicentre study is carried out with an enrolment to last approximately 9 months.

An open-label, phase I/IIa trial, dose-escalation study with a 3 + 3 cohort design is carried out to determine the recommended dose of E4 for the treatment of patients with advanced breast cancer. After completing the phase I part of the study (i.e. 4 weeks of treatment), patients receive further treatment for 8 weeks at their individual phase I dose level (phase IIa part of the study). Patients are treated for 12 weeks. Treatment continues after 12 weeks of treatment until the patient is withdrawn from the study either due to unacceptable adverse events (AEs) or disease progression. These patients are followed up according to clinical practice including radiological tumour assessments.

**Subject population:** In total at least 9 patients with ER+ locally advanced and/or metastatic breast cancer having exhausted all treatment options are enrolled in the study. Patients are at least 5 years postmenopausal and have failed anti-estrogen treatment with tamoxifen and aromatase inhibitor because the cancer has become resistant to treatment with tamoxifen or aromatase inhibitor, or because the patient has discontinued treatment due to unacceptable side-effects. No treatment with fulvestrant is allowed within 6 months prior to the start of the E4 treatment.

### Study objectives and variables:

*Primary Objective:* to assess dose-limiting toxicity (DLT) of E4 treatment to estimate the maximally recommended dose in patients with advanced breast cancer. This is assessed by following the number of patients with a DLT in phase I part of the study (i.e. 4 weeks of treatment).

### Secondary Objectives:

- to assess quality of life in advanced breast cancer patients treated with E4, using specific questions on estrogen deficiency symptoms;
- to assess preliminary anti-tumour response of E4 in patients with advanced breast cancer, according to RECIST 1.1 at the end of the study (i.e. after 12 weeks);
- to assess the trough (Cmin) levels of E4 in patients with advanced breast cancer, by measuring E4 plasma concentrations before dosing at different time points during the study.

### Safety Objective:

- to evaluate the safety profile of E4 in patients with advanced breast cancer by:
   o Performing routine laboratory parameters;
   o Performing physical examination;
   o Measuring vital signs;
   o Measuring electrocardiograms (ECGs);
   o Recording and monitoring of incidence and severity of clinical AEs and SAEs;
   o Radiological tumour assessments.

### Design of the study:

The phase I part of the study follows the traditional 3 + 3 design with inter-patient dose escalation to select the recommended dose in patients with advanced breast cancer (recommended dose is the dose that causes a DLT in ≤17 % of the patients). Patients are enrolled in cohorts at sequentially rising dose levels of E4. A Safety Review Committee, set up prior to the start of the study, reviews safety and toxicity data for each cohort, and decides on how to proceed with the dose escalation.

Patients are treated in cohorts of three patients, each receiving the same dose level. If none of the three patients show a DLT at that dose level, the next cohort of three patients receives the next higher dose.

Otherwise, a second cohort of three patients is treated at the same dose level. If exactly one out of the six patients treated at a particular dose exhibits a DLT, the trial continues at the next higher dose level. If two or more patients out of the six exhibit a DLT at a certain dose level, the escalation stops at that level. This dose level is considered to be a non-tolerable dose level. The recommended dose is then defined as the next lower dose level.

In case two or more events of DLTs occur in the same patient among a cohort of three patients, it is up to the discretion of the Safety Review Committee to determine whether a non-tolerable dose has been reached. Dose escalation starts at 20 mg, and escalates in steps of 20 mg up to a dose of maximally 60 mg of E4. Patients not completing 4 weeks of treatment (phase I) for reasons other than DLT are considered to be un-evaluable for toxicity and are replaced.

After completing the phase I part of the study (i.e. 4 weeks of treatment), patients receive further treatment for 8 weeks at their individual phase I dose level (phase Ila part of the study). The phase Ila part of the study includes explorative efficacy assessments to evaluate potential anti-tumour effects of E4 by RECIST criteria based on evaluation of pre-and end of treatment imaging.

### Test Product. Dose, and Mode of Administration:

Treatment is escalated in doses of 20 mg, 40 mg and 60 mg E4 across cohorts according to the 3+3 design until the non-tolerable dose is determined or the maximum anticipated dose (MAD) of 60 mg is reached. Patients are treated once daily with an oral administration of E4.

Patients who complete the two study phases (i.e. 12 weeks) and did not show progression of the disease are offered further E4 treatment at the investigators' discretion. These patients are followed up according to clinical practice including radiological tumour assessments. Treatment may continue until the patient is withdrawn either due to unacceptable AEs or disease progression.

### Statistical Methods:

Statistical analyses are performed on four populations of patients: **Full Analysis Set (FAS)**: All the subjects who met eligibility criteria and signed the Informed consent form (ICF). **Intent-to-treat (ITT):** The ITT population consists of all the patients who received at least one dose of the study medication. The ITT is used for analyses of safety.

### Example 4

A female breast cancer patient, 71 years old, who had previously received three different types of estrogen activity suppressors (letrozole, tamoxifen and anastrozole) participated in the study that is described in Example 3. She had discontinued all estrogen activity suppressor treatments due to unacceptable side effects (including increase of depression).

Patient had not been treated with chemotherapy as she was unwilling to accept the adverse effect on QOL related to the chemotherapy treatment.

At the start of the E4 treatment, the patient had progressive disease. The patient was treated with 20 mg E4 per day (lowest and starting dose in the study). She tolerated the treatment very well. Only one AE considered to be related to the E4 treatment was reported for the patient during 12 weeks of treatment. The patient experienced breast tenderness with mild intensity. She reported to feel much better than before the start of the E4 treatment, so the treatment had an advantageous effect on her QOL. E4 also had a favourable impact on tumour progression as the tumour burden remained stable during treatment. The patient decided to continue with the same dose of E4 after completion of the 12 week study period.

## Claims

1. Estetrol component for use in the treatment of advanced estrogen receptor positive breast cancer in a subject who has been treated with an estrogen activity suppressor selected from a selective estrogen receptor modulator (SERM), an aromatase inhibitor and an anti-estrogen, said treatment comprising administration of the estetrol component to the subject within 12 weeks after the treatment with an estrogen activity suppressor has been discontinued, said estetrol component being selected from estetrol, prodrugs of estetrol in the form of estetrol derivatives wherein the hydrogen atom of at least one of the hydroxyl groups has been substituted by an acyl radical of a hydrocarbon carboxylic, sulfuric acid, sulfonic acid or sulfamic acid of 1-25 carbon atoms, and combinations thereof.

2. Estetrol component for use in the treatment according to claim 1, wherein the treatment comprises the administration of the estetrol component for a duration of at least 8 weeks, preferably at least 24 weeks, more preferably at least 1 year.

3. Estetrol component for use in the treatment according to claim 1 or 2, wherein the treatment comprises the administration of the estetrol component to a subject whose breast cancer has acquired resistance to said estrogen activity suppressor.

4. Estetrol component for use in the treatment according to claim 1 or 2, wherein the treatment comprises the administration of the estetrol component to an oophorectomized or post-menopausal female subject whose treatment with an estrogen activity suppressor was discontinued following the occurrence of unacceptable side effects.

5. Estetrol component for use in the treatment according to claims 1-4, wherein the tumour burden is monitored during the treatment, and administration of a chemotherapy agent is commenced when the monitoring shows that the tumour burden has increased.

6. Estetrol component for use in the treatment according to claim 5, wherein administration of the estetrol component is continued during administration of the chemotherapy agent.

7. Estetrol component for use in the treatment according to any one of the previous claims wherein the treatment comprises uninterrupted administration of doses of the estetrol component during a period of at least 2 weeks in dosages equivalent to a daily oral dosage of 10 to 80 mg estetrol, more preferably of 20 to 60 mg estetrol.

8. Estetrol component for use in the treatment according to any one of the preceding claims, wherein the treatment comprises oral, sublingual, buccal or sublabial administration of the estetrol component.

9. Estetrol component for use in the treatment according to claim 8, wherein the treatment comprises oral administration of the estetrol component.

10. Estetrol component for use in the treatment according to any one of the previous claims, wherein the subject has not been treated with fulvestrant in the 6-months period preceding administration of the estetrol component.

11. Estetrol component for use in the treatment according to any one of the previous claims, wherein the subject is a post-menopausal human female or an oophorectomized human female.

12. Estetrol component for use in the treatment according to any one of the preceding claims, wherein the estetrol component is estetrol.

13. An oral dosage unit comprising an oral chemotherapy drug selected from capecitabine, cyclophosphamide, vinorelbine, methotrexate and combinations thereof, together with 10-80 mg estetrol component, said estetrol component being selected from estetrol, prodrugs of estetrol in the form of estetrol derivatives wherein the hydrogen atom of at least one of the hydroxyl groups has been substituted by an acyl radical of a hydrocarbon carboxylic, sulfuric acid, sulfonic acid or sulfamic acid of 1-25 carbon atoms, and combinations thereof.

14. A kit-of-parts comprising at least one dosage unit containing a chemotherapy drug selected from gemcitabine, docetaxel, paclitaxel, albumin-bound paclitaxel, cisplatin, carboplatin, doxorubicin, liposomal doxorubicin, epirubicin, eribulin, ixabepilone, cyclophosphamide, vinorelbine and combinations thereof, and a plurality of oral dosage units containing 10-100 mg estetrol component, said estetrol component being selected from estetrol, prodrugs of estetrol in the form of estetrol derivatives wherein the hydrogen atom of at least one of the hydroxyl groups has been substituted by an acyl radical of a hydrocarbon carboxylic, sulfuric acid, sulfonic acid or sulfamic acid of 1-25 carbon atoms, and combinations thereof.

## Patentansprüche

1. Estetrol-Komponente zur Verwendung bei der Behandlung von fortgeschrittenem Östrogenrezeptor-positivem Brustkrebs bei einem Subjekt, das mit einem Östrogenaktivitätssuppressor behandelt wurde, der aus einem selektiven Östrogenrezeptormodulator (SERM), einem Aromataseinhibitor und einem Anti-Östrogen ausgewählt ist, wobei die Behandlung die Verabreichung der Estetrol-Komponente an das Subjekt innerhalb von 12 Wochen, nachdem die Behandlung mit einem Östrogenaktivitätssuppressor abgebrochen wurde, umfasst, wobei die Estetrol-Komponente aus Estetrol, Prodrugs von Estetrol in Form von Estetrol-Derivaten, wobei das Wasserstoffatom von mindestens einer der Hydroxylgruppen durch ein Acylradikal einer Kohlenwasserstoffcarbonsäure, Schwefelsäure, Sulfonsäure oder Sulfaminsäure mit 1-25 Kohlenstoffatomen substituiert wurde, und Kombinationen davon ausgewählt ist.

2. Estetrol-Komponente zur Verwendung bei der Behandlung nach Anspruch 1, wobei die Behandlung die Verabreichung der Estetrol-Komponente für eine Dauer von mindestens 8 Wochen, vorzugsweise mindestens 24 Wochen, bevorzugter mindestens 1 Jahr, umfasst.

3. Estetrol-Komponente zur Verwendung bei der Behandlung nach Anspruch 1 oder 2, wobei die Behandlung die Verabreichung der Estetrol-Komponente an ein Subjekt umfasst, dessen Brustkrebs eine Resistenz gegen den Östrogenaktivitätssuppressor erworben hat.

4. Estetrol-Komponente zur Verwendung bei der Behandlung nach Anspruch 1 oder 2, wobei die Behandlung die Verabreichung der Estetrol-Komponente an ein oophorektomiertes oder postmenopausales weibliches Subjekt umfasst, dessen Behandlung mit einem Östrogenaktivitätssuppressor nach dem Auftreten von inakzeptablen Nebenwirkungen abgebrochen wurde.

5. Estetrol-Komponente zur Verwendung bei der Behandlung nach den Ansprüchen 1-4, wobei die Tumorbelastung während der Behandlung überwacht wird und die Verabreichung eines Chemotherapeutikums begonnen wird, wenn die Überwachung zeigt, dass die Tumorbelastung zugenommen hat.

6. Estetrol-Komponente zur Verwendung bei der Behandlung nach Anspruch 5, wobei die Verabreichung der Estetrol-Komponente während der Verabreichung des Chemotherapeutikums fortgesetzt wird.

7. Estetrol-Komponente zur Verwendung bei der Behandlung nach einem der vorhergehenden Ansprüche, wobei die Behandlung eine ununterbrochene Verabreichung von Dosen der Estetrol-Komponente während eines Zeitraums von mindestens 2 Wochen in Dosierungen umfasst, die einer täglichen oralen Dosierung von 10 bis 80 mg Estetrol, bevorzugter von 20 bis 60 mg Estetrol, entsprechen.

8. Estetrol-Komponente zur Verwendung bei der Behandlung nach einem der vorhergehenden Ansprüche, wobei die Behandlung eine orale, sublinguale, bukkale oder sublabiale Verabreichung der Estetrol-Komponente umfasst.

9. Estetrol-Komponente zur Verwendung bei der Behandlung nach Anspruch 8, wobei die Behandlung eine orale Verabreichung der Estetrol-Komponente umfasst.

10. Estetrol-Komponente zur Verwendung bei der Behandlung nach einem der vorhergehenden Ansprüche, wobei das Subjekt in der 6-Monats-Periode, die der Verabreichung der Estetrol-Komponente vorausgeht, nicht mit Fulvestrant behandelt wurde.

11. Estetrol-Komponente zur Verwendung bei der Behandlung nach einem der vorhergehenden Ansprüche, wobei das Subjekt eine postmenopausale menschliche Frau oder eine oophorektomierte menschliche Frau ist.

12. Estetrol-Komponente zur Verwendung bei der Behandlung nach einem der vorhergehenden Ansprüche, wobei die Estetrol-Komponente Estetrol ist.

13. Orale Dosierungseinheit, umfassend ein orales Chemotherapiemedikament, das aus Capecitabin, Cyclophosphamid, Vinorelbin, Methotrexat und Kombinationen davon ausgewählt ist, zusammen mit 10-80 mg Estetrol-Komponente, wobei die Estetrol-Komponente aus Estetrol, Prodrugs von Estetrol in Form von Estetrol-Derivaten, wobei das Wasserstoffatom von mindestens einer der Hydroxylgruppen durch ein Acylradikal einer Kohlenwasserstoffcarbonsäure, Schwefelsäure, Sulfonsäure oder Sulfaminsäure mit 1-25 Kohlenstoffatomen substituiert wurde, und Kombinationen davon ausgewählt ist.

14. Satz-von-Teilen, umfassend mindestens eine Dosierungseinheit, die ein Chemotherapiemedikament enthält, das aus Gemcitabin, Docetaxel, Paclitaxel, albumingebundenem Paclitaxel, Cisplatin, Carboplatin, Doxorubicin, liposomalem Doxorubicin, Epirubicin, Eribulin, Ixabepilon, Cyclophosphamid, Vinorelbin und Kombinationen davon ausgewählt ist, und mehrere orale Dosierungseinheiten, die 10-100 mg Estetrol-Komponente enthalten, wobei die Estetrol-Komponente aus Estetrol, Prodrugs von Estetrol in Form von Estetrol-Derivaten, wobei das Wasserstoffatom von mindestens einer der Hydroxylgruppen durch ein Acylradikal einer Kohlenwasserstoffcarbonsäure, Schwefelsäure, Sulfonsäure oder Sulfaminsäure mit 1-25 Kohlenstoffatomen substituiert wurde, und Kombinationen davon ausgewählt ist.

## Revendications

1. Composant estétrol à utiliser dans le traitement du cancer du sein positif aux récepteurs d'œstrogènes avancé chez un sujet qui a été traité avec un suppresseur d'activité œstrogénique choisi parmi un modulateur sélectif de récepteurs d'œstrogènes (SERM), un inhibiteur d'aromatase et un anti-œstrogène, ledit traitement comprenant l'administration du composant estétrol au sujet dans les 12 semaines après que le traitement avec un suppresseur d'activité œstrogénique a été interrompu, ledit composant estétrol étant choisi parmi l'estétrol, des promédicaments de l'estétrol sous la forme de dérivés d'estétrol dans lesquels l'atome d'hydrogène d'au moins un des groupes hydroxyle a été substitué par un radical acyle d'un acide carboxylique hydrocarboné, d'un acide sulfurique, d'un acide sulfonique ou d'un acide sulfamique de 1 à 25 atomes de carbone, et des combinaisons de ceux-ci.

2. Composant estétrol à utiliser dans le traitement selon la revendication 1, dans lequel le traitement comprend l'administration du composant estétrol pendant une durée d'au moins 8 semaines, de préférence d'au moins 24 semaines, de manière plus préférée d'au moins 1 an.

3. Composant estétrol à utiliser dans le traitement selon la revendication 1 ou 2, dans lequel le traitement comprend l'administration du composant estétrol à un sujet dont le cancer du sein a acquis une résistance audit suppresseur d'activité œstrogénique.

4. Composant estétrol à utiliser dans le traitement selon la revendication 1 ou 2, dans lequel le traitement comprend l'administration du composant estétrol à un sujet féminin ayant subi une oophorectomie ou post-ménopausé dont le traitement avec un suppresseur d'activité œstrogénique a été interrompu après l'apparition d'effets secondaires inacceptables.

5. Composant estétrol à utiliser dans le traitement selon les revendications 1 à 4, dans lequel la charge tumorale est surveillée pendant le traitement, et l'administration d'un agent de chimiothérapie est commencée lorsque la surveillance montre que la charge tumorale a augmenté.

6. Composant estétrol à utiliser dans le traitement selon la revendication 5, dans lequel l'administration du composant estétrol est poursuivie pendant l'administration de l'agent de chimiothérapie.

7. Composant estétrol à utiliser dans le traitement selon l'une quelconque des revendications précédentes, dans lequel le traitement comprend une administration ininterrompue de doses du composant estétrol pendant une période d'au moins 2 semaines à des doses équivalentes à une dose orale quotidienne comprise entre 10 à 80 mg d'estétrol, de manière plus préférée comprise entre 20 et 60 mg d'estétrol.

8. Composant estétrol pour une utilisation dans le traitement selon l'une quelconque des revendications précédentes, dans lequel le traitement comprend une administration orale, sublinguale, buccale ou sous-labiale du composant estétrol.

9. Composant estétrol à utiliser dans le traitement selon la revendication 8, dans lequel le traitement comprend une administration orale du composant estétrol.

10. Composant estétrol à utiliser dans le traitement selon l'une quelconque des revendications précédentes, dans lequel le sujet n'a pas été traité avec du fulvestrant dans la période de 6 mois précédant l'administration du composant estétrol.

11. Composant estétrol à utiliser dans le traitement selon l'une quelconque des revendications précédentes, dans lequel le sujet est une femme humaine post-ménopausée ou une femme humaine ayant subi une oophorectomie.

12. Composant estétrol à utiliser dans le traitement selon l'une quelconque des revendications précédentes, dans lequel le composant estétrol est l'estétrol.

13. Unité posologique orale comprenant un médicament de chimiothérapie oral choisi parmi la capécitabine, le cyclophosphamide, la vinorelbine, le méthotrexate et des combinaisons de ceux-ci, comprenant de 10 à 80 mg de composant estétrol, ledit composant estétrol étant choisi parmi l'estétrol, des promédicaments d'estétrol sous la forme de dérivés d'estétrol dans lesquels l'atome d'hydrogène d'au moins l'un des groupes hydroxyle a été substitué par un radical acyle d'un acide carboxylique hydrocarboné, d'un acide sulfurique, d'un acide sulfonique ou d'un acide sulfamique de 1 à 25 atomes de carbone, et des combinaisons de ceux-ci.

14. Kit de pièces comprenant au moins une unité posologique contenant un médicament de chimiothérapie choisi parmi la gemcitabine, le docétaxel, le paclitaxel, le paclitaxel lié à l'albumine, le cisplatine, le carboplatine, la doxorubicine, la doxorubicine liposomale, l'épirubicine, l'éribuline, l'ixabépilone, le cyclophosphamide, la vinorelbine et des combinaisons de ceux-ci, et une pluralité d'unités posologiques orales contenant entre 10 à 100 mg de composant estétrol, ledit composant estétrol étant choisi parmi l'estétrol, des promédicaments d'estétrol sous la forme de dérivés d'estétrol dans lesquels l'atome d'hydrogène d'au moins l'un des groupes hydroxyle a été substitué par un radical acyle d'un acide carboxylique hydrocarboné, d'un acide sulfurique, d'un acide sulfonique ou d'un acide sulfamique de 1 à 25 atomes de carbone, et des combinaisons de ceux-ci.
